# EUROPÄISCHE PATENTSCHRIFT

(11) **EP 2 194 004 B1**
(45) Veröffentlichungstag und Bekanntmachung des Hinweises auf die Patenterteilung: **20.02.2013**
(21) Anmeldenummer: 10157386.3
(22) Anmeldetag: 24.04.2007
(51) Int. Cl.: B65D 79/02, G01N 33/46, G01N 31/22

(54) **Paneelverpackung mit Indikator**
Wainscot panel package with indicator
Conditionnement de lambris avec indicateur

(30) Priorität: 02.05.2006 DE 102006020619
(43) Veröffentlichungstag der Anmeldung: 09.06.2010
(62) Teilanmeldung aus: 07728443.8
(73) Patentinhaber: Fritz Egger GmbH & Co. OG, 3105 Unterradlberg (AT)
(72) Erfinder: Steinwender, Martin, 2380, Perchtoldsdorf (AT)
(74) Vertreter: Cohausz & Florack

(56) Entgegenhaltungen:
- EP-A1- 1 450 158
- WO-A-02/071056
- DE-A1- 10 128 807
- DE-U1-202004 017 254
- DE-U1-202004 017 254
- GB-A- 2 258 918
- US-A- 1 690 672

## Beschreibung

Die Erfindung betrifft eine Paneelverpackungsanordnung mit einer Umhüllung und mit mindestens einem von der Umhüllung aufgenommenen Paneel. Eine Paneelverpackung und ein entsprechendes Paneel sind aus der DE 20 2004 017 254 U1 bekannt.

Paneelverpackungen sind seit langem bekannt. Dabei werden vielfältige Ausführungsformen in Bezug auf die Materialien, Größe, geometrische Form und optischer Ausgestaltung der Umhüllungen gewählt. Insbesondere können Umhüllungen aus einem Kartonmaterial hergestellt sein, von denen ein oder mehrere Paneele aufgenommen werden.

Weiterhin ist es bekannt, Paneele mit Umhüllungen aus Verpackungsfolien zu verpacken. Auch eine Kombination eines Verpackungskartons sowie einer Verpackungsfolie ist aus dem Stand der Technik hinreichend bekannt.

Bei den bekannten Paneelverpackungen ist es von Nachteil, dass die Handhabung der Paneelverpackungen bzw. der Paneele durch eingeschränkte Informationen erschwert ist.

Zum einen ist dem Verleger bzw. Endverbraucher nur ein geringer Informationsgehalt über die Paneelverpackungen und/oder die Paneele ab Auslieferung zugänglich. Auch für den Hersteller weisen die bekannten Paneelverpackungen diese Nachteile auf, da er, sobald die Paneelverpackung seinen Einflußbereich verlassen hat, keine Möglichkeit besitzt Informationen - abgesehen von der derzeitigen Lagerposition - der Paneelverpackung und/oder der Paneele zu erlangen.

Zudem sind aus der WO 00/75417 und der WO 03/038777 Böden bekannt, bei denen Sensoren vorgesehen sind, die eine Beanspruchung des Bodens an eine Datenverarbeitungsanlage übermitteln können. Die Übermittlung kann dabei durch fest verbundene Leitungen oder über Funk realisiert sein. Solche Böden werden im verlegten Zustand zur Analyse von Beanspruchungen und zum Regeln bestimmter Zustände in Abhängigkeit der Beanspruchungen verwendet. Beispielsweise läßt sich so ein Boden realisieren, der beim Betreten einen Alarm auslöst oder eine Klimaanlage regelt. Bei den bekannten Böden ist es von Nachteil, dass die Sensoren aufwändig herzustellen sind und weitere Geräte zur Auswertung der Sensoren benötigt werden.

Die WO 02/071056 A1 offenbart ein Bauteil, insbesondere einen Fensterrahmen, mit einem Feuchteindikator. Ebenfalls ein Feuchteindikator in einem Holzbauteil ist aus der US 1,690,672 und der GB 2 258 918 A, wobei es sich hier um einen Holzbalken mit Indikator handelt, bekannt. Die DE 101 28 807 A1 offenbart einen Indikator, der dazu dient, die durch Nässe oder Feuchtigkeit hervorgerufene endgültige Unbrauchbarkeit eines Guts, beispielsweise einer Spanplatte oder eines Bretts, unmittelbar oder auf einem Packmittel durch irreversible Verfärbung anzuzeigen. Die EP 1 450 158 A1 betrifft einen Feuchteindikator für Holzscheite, Schnitzel und Sägemehl, um die Holzfeuchte für die Verwendung eines Holzes in einem Holzofen zu bestimmen.

Der Erfindung liegt somit das technische Problem zugrunde, die Handhabung von Paneelverpackungen durch größeren Informationsumfang zu verbessern und zu erleichtern.

Das zuvor aufgezeigte technische Problem wird erfindungsgemäß durch die Merkmale von Patentanspruch 1 gelöst.

Durch Vorsehen von Indikatoren wird die Möglichkeit gegeben en wird, Informationen über eine Temperatur- und/oder Feuchtigkeits- Beanspruchung direkt, insbesondere mit dem bloßen Auge und ohne weitere Hilfsmittel, individuell an der Paneelverpackung abzulesen.

Es sind mehrere Indikatoren vorgesehen, auf der Außen- und Innenseite der Verpackungsfolie. Wird erfindungsgemäß ein weiterer Indikator an der Innenseite der Verpackungsfolie angebracht, so kann die Beanspruchung nach dem Öffnen der Paneelverpackung angezeigt werden. So kann eine Konditionierung der verschlossenen Paneelverpackung in dem Raum, in dem die Paneele verlegt werden sollen, an den angebrachten Indikatoren abgelesen werden. Es kann dann vorgeschrieben sein, dass ein Öffnen der Paneelverpackung und anschließendes Verlegen erst nach einem Angleichen der Indikatoren zulässig ist.

Als Indikatoren eignen sich beispielsweise alle Indikatoren, die in Salzen, die zur Trocknung Anwendung finden, verwendet werden, um zu signalisieren, ob die Salze bereits Wasser aufgenommen haben, oder ob sie noch zur Trocknung eingesetzt werden können.

Eine Änderung des Erscheinungsbildes kann durch eine Änderung der Farbe des Indikators ermöglicht werden. Weiterhin kann der Indikator verschiedene Felder aufweisen, die beispielsweise zwei verschiedene Zustände annehmen können. So kann eine Änderung des Erscheinungsbildes eine Änderung der Anzahl der Felder eines gleichen Zustandes sein.

Der Indikator wird im Folgenden näher beschrieben.

Erfindungsgemäß ändern die Indikatoren, ständig in Abhängigkeit von der mindestens einen Beanspruchung ihr Erscheinungsbild. So kann jederzeit festgestellt werden, in welchem Zustand sich die Paneelverpackung bzw. die Paneele zu einem bestimmten Zeitpunkt befinden. In Abhängigkeit von diesem Zustand können dann Entscheidungen bezüglich der Verlegbarkeit der Paneele getroffen werden und die Handhabung der Paneelverpackung bzw. der Paneele erheblich vereinfacht werden.

Dabei ist die mindestens eine Beanspruchung eine durch Temperatur und/oder Feuchtigkeit bedingte Beanspruchung. So kann ein Unter- bzw. Überschreiten von klimatischen Grenzwerten bezüglich der zulässigen Temperatur und/oder Feuchtigkeit während des Transports sichtbar gemacht werden. Insbesondere kann dies bei Containertransporten über große Distanzen von großem Vorteil sein.

Der Indikator kann die Einhaltung der Verlegevorschriften anzeigen. Er kann so gestaltet sein, dass an ihm abgelesen werden kann, ob die Paneelverpackung beispielsweise zwischen 15°C und 20°C, insbesondere bei mindestens 18°C und zwischen 30% und 80%, insbesondere zwischen 40% bis 70% Luftfeuchtigkeit über einen bestimmten Zeitraum von Tagen, insbesondere 24 oder 48 Stunden vorkonditioniert wurde.

Weiterhin kann ein Indikator vorgesehen sein, der eine durch mechanische Spannung bedingte Beanspruchung anzeigen kann. Beispielsweise kann sich ein solcher Indikator bei einer zu großen Belastung der Paneelverpackung verfärben. Auch kann ein solcher Indikator als verkapseltes System ausgeführt sein und an einem oder allen Paneelen vorgesehen sein. Bei einer zu großen Belastung setzen die Kapseln einen Farbstoff frei. So können kleinste Beschädigungen der Paneele, beispielsweise der Kantenbereiche, sichtbar gemacht werden und der Verleger kann die entsprechenden Paneele zum Verlegen in den Randbereichen sowie durch Abtrennen der beschädigten Kanten verwenden.

Auch kann die mindestens eine Beanspruchung eine durch Licht bedingte Beanspruchung sein. So kann an dem Indikator abgelesen werden, ob die Paneelverpackung und insbesondere ein Paneel einer zu starken Belastung durch Licht ausgesetzt wurde. Dadurch kann verhindert werden, dass ein etwaiges verblassen des Dekors eines Paneels erst nach dem Verlegen festgestellt wird.

Besonders vorteilhaft kann der Indikator an mindestens einem Befestigungselement angebracht sein.

Dabei kann das mindestens eine Befestigungselement eine Verpackungsfolie der Umhüllung sein. Da ein Indikator von außen auf der Paneelverpackung aufgebracht ist, kann er zur Anzeige der Belastungen, die von außen auf die Paneelverpackung wirken, verwendet werden. So kann ein Indikator auch an der Außenseite des Bodens der Paneelverpackung vorgesehen sein. Wird die Paneelverpackung dann eine bestimmte Zeit auf dem Boden, auf dem die Paneele verlegt werden sollen, belassen, kann im Anschluss anhand des Indikators abgelesen werden, ob der Boden die nötige Trockenheit aufweist.

Ebenso kann das mindestens eine Befestigungselement ein Verpackungskarton sein. Besonders bei Verwendung einer Paneelverpackung, die aus einer Verpackungsfolie und einem Verpackungskarton besteht ist dies vorteilhaft, da so nach Entfernen der Verpackungsfolie die Paneele zum Konditionieren in dem Verpackungskarton verbleiben können, wobei direkt an dem Verpackungskarton der Zustand der Paneele durch den Indikator angezeigt werden kann.

Eine kostengünstige Möglichkeit ist es, wenn das mindestens eine Befestigungselement ein Einleger bzw. ein Etikett ist. Dann können die Indikatoren in großen Stückzahlen hergestellt werden und an den Paneelverpackungen befestigt werden.

Eine Ausführungsform ist **dadurch gekennzeichnet, dass** der Indikator eine Abdeckung aufweist. Insbesondere kann es sich dabei um eine den Indikator deaktivierende Abdeckung handeln. Erst nachdem die Abdeckung entfernt worden ist, passt sich der Indikator an die Umgebung an. So könnte ein Indikator an der Innenseite der Verpackungsfolie vorgesehen sein, der von einem zusätzlichen Verpackungskarton der Paneele oder durch ein Paneel selbst deaktiviert ist.

Besonders bevorzugt ist es, wenn mindestens eine das Erscheinungsbild des Indikators erläuternde Skala vorgesehen ist. Eine Skala kann dabei unmittelbar neben dem Indikator vorgesehen sein. Beispielsweise kann eine Skala auf der Außenseite und/oder Innenseite der Verpackungsfolie vorgesehen sein. Es kann sich um eine Vergleichsskala handeln, die bestimmte Farben und deren Bedeutung in Bezug auf die Temperatur und/oder Feuchtigkeit des Indikators erläutert. Die Skala kann aufgedruckt werden.

Es ist auch möglich, einen Indikator auf das Befestigungselement aufzudrucken. So kann durch eine flexible Produktion je nach Bedarf eine Paneelverpackung mit einem Indikator versehen werden.

Es können gleiche Indikatoren mit gleichen oder unterschiedlichen Skalen verwendet werden. Beispielsweise kann der gleiche Indikator am Boden und an der Seite der Paneelverpackung vorgesehen sein. Am Boden könnte eine Skala "Bodenkondition", an der Seite eine Skala "Umgebungskondition" angebracht sein. Auch können je nach Funktion unterschiedliche Indikatoren vorgesehen sein Insbesondere kann die Zusammensetzung des Indikators an den jeweils anzuzeigenden Zustand angepasst sein.

Die für einen Verleger bzw. bei der Verlegung durch das ständige Ändern des Erscheinungsbildes des Indikators beispielsweise anzeigbaren Beanspruchungen können dabei mittelbar zur Anzeige von weiteren Zuständen der Paneele dienen.

Durch entsprechende Ausgestaltung kann ein Indikator, der eine Beanspruchung oder eine Kombination von Beanspruchungen durch Temperatur und/oder Feuchte bzw. Zusätlich mechanischer Beanspruchung anzeigt, dazu verwendet werden, eine Aussage über das Risiko von Peaking (Kantenhochzug), Verzug, Verwerfungen, Längendehnungen, Bananenproblem, Winkelproblem oder sonstiger Probleme, die durch nicht ausreichende Konditionierung der in der Paneelverpackung befindlichen Paneele entstehen können, zu treffen.

Bei den mittelbar festzustellenden Beanspruchungen seien, jedoch nicht abschließend, genannt: zu feuchter Untergrund, zu feuchter Transport, zu heiße Vorlauftemperatur einer Bodenheizung, zu feuchtes/trockenes Umgebungsklima, zu feuchte Reinigung oder Wasserschäden sowie mechanische Beschädigungen durch den Endverbraucher.

Die vorliegende Erfindung soll im Folgenden anhand spezieller Ausführungsbeispiele sowie der beigefügten Zeichnung näher erläutert werden. Die Zeichnung zeigt in
- Fig. 1: eine Paneelverpackung mit einem an der Verpackungsfolie befestigten Indikator,
- Fig. 2: eine geöffnete Paneelverpackung mit einem an der Verpackungsfolie und einem an dem Verpackungskarton befestigten Indikator, jeweils mit einer erläuternden Skala und in
- Fig. 3: eine Paneelverpackung mit einem an dem Verpackungskarton befestigten Indikator sowie einem an einem Paneel befestigten Indikator.

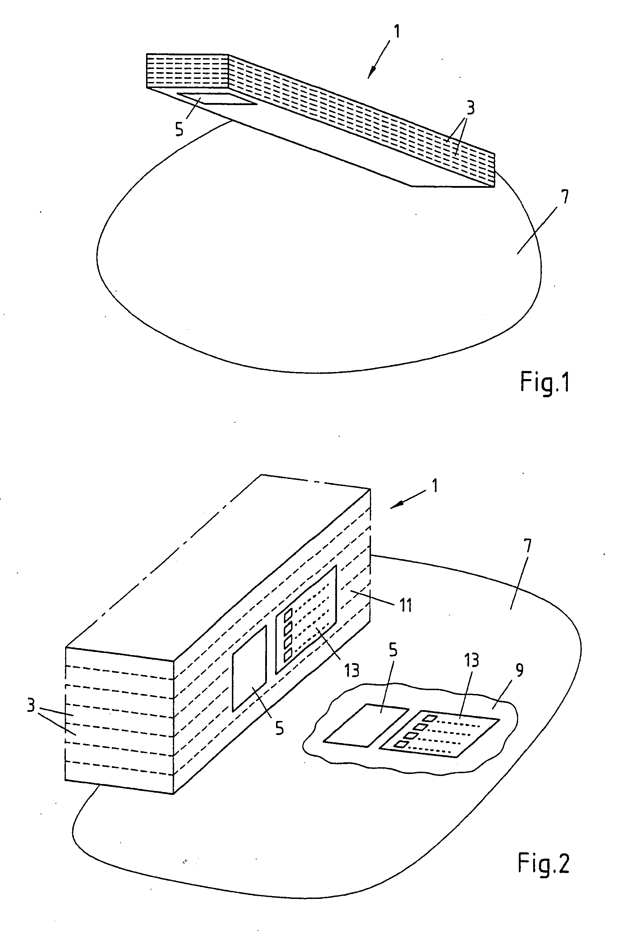

Fig. 1 zeigt eine Paneelverpackung 1 mit mehreren von der Paneelverpackung 1 aufgenommenen Paneelen 3 und mit einem Indikator 5 zur Anzeige von einer Beanspruchung durch Temperatur und Feuchtigkeit, wobei der Indikator 5 ständig in Abhängigkeit von der Beanspruchung sein Erscheinungsbild ändert. Dabei kann die Umhüllung der Paneelverpackung aus einer Verpackungsfolie bestehen.

Eine solche Ausführungsform ist besonders geeigne, um jederzeit ein etwaiges Unter- oder Überschreiten zulässiger Grenzwerte anzuzeigen.

Alternativ kann der Indikator 5 für eine Auflage auf einem Boden 7 vorgesehen sein. Durch Belassen der Paneelverpackung 1 auf dem Boden 7 für einen vorgeschriebenen Zeitraum kann der Indikator 5 dazu verwendet werden, eine Aussage über die Trockenheit des Bodens 7 zu treffen.

Fig. 2 zeigt eine geöffnete Paneelverpackung 1, mit Paneelen 3. Die Umhüllung wird in dem in Fig. 2 gezeigten Ausführungsbeispiel aus einer Verpackungsfolie 9 und einem Verpackungskarton 11 gebildet. Vor dem Öffnen der Verpackungsfolie 9 war der Verpackungskarton 11 von der Verpackungsfolie 9 umschlossen. Die Verpackungsfolie 9 sowie der Verpackungskarton 11 weisen jeweils Indikatoren 5 und eine jeweils einen Indikator 5 erläuternde Skala 13 auf.

Die Skala 13 kann beispielsweise vier Felder verschiedener Farbe aufweisen. Zusätzlich ist neben jedem Feld ein die Farbe erläuternder Text vorgesehen.

Die in Fig. 2 gezeigte Ausführungsform kann dazu verwendet werden, dass zunächst die Paneelverpackung 1 im geschlossenen Zustand über einen bestimmten Zeitraum von Tagen, insbesondere 24 oder 48 Stunden vorkonditioniert wird. Dabei nimmt der an der Verpackungsfolie 9 angebrachte Indikator 5 - der an der im verpackten Zustand nach außen gerichteten Fläche der Verpackungsfolie 9 angebracht ist - eine bestimmte Farbe an, die auf der Skala 13 als verlegetauglich erläutert wird.

Es kann dann vorgeschrieben sein, dass ein Öffnen der Paneelverpackung 1 und ein Verlegen der Paneele 3 nicht vor dem Angleichen der beiden Indikatoren 5 erfolgen darf. Somit kann sichergestellt werden, dass sich die Paneele 3 ausreichend lange an die Umgebungsbedingungen anpassen konnten.

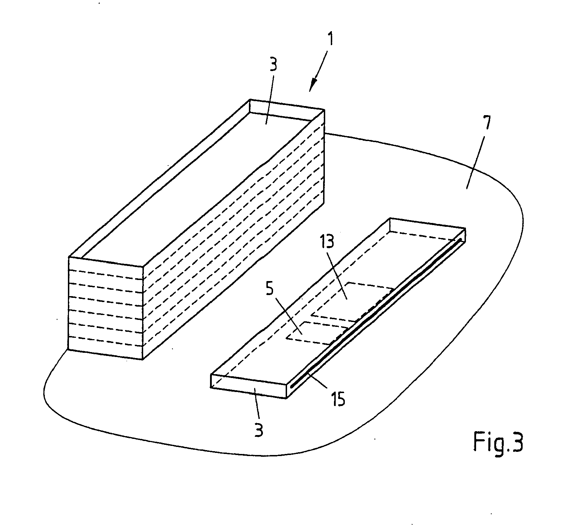

Fig. 3 zeigt eine geöffnete Paneelverpackung 1, aus der ein Paneel 3 entnommen wurde. Das Paneel 3 weist an seiner Unterseite einen Indikator 5 auf. Insbesondere kann der Indikator 5 an einer Trittschalldämmung befestigt sein. Zusätzlich ist an den Längskanten des Paneels 3 ein Indikator 15 vorgesehen. Der Indikator 5 kann, wie zuvor erläutert, zur Anzeige der Feuchtigkeit des Bodens 7 verwendet werden. Ein Teil der Skala 13, die in Fig. 3 ebenfalls an der Unterseite des Paneels 3 vorgesehen ist, dient zur Erläuterung des Indikators 5.

Der Indikator 15 kann verkapselt ausgeführt sein, so dass er bei zu großer mechanischer Beanspruchung der Längskanten eine auf der Skala 13 erläuterte Verfärbung aufweist.

Ebenso kann der Indikator 15 derart ausgeführt sein, dass er ein Peaking der Längskante oder einen Verzug des Paneels 3 durch Änderung seines Erscheinungsbildes anzeigen kann.

Die Erfindung ist nicht auf die zuvor genannten Ausführungsbeispiele beschränkt. Insbesondere kann der Indikator mittels eines Einlegers oder Etiketts mit der Verpackung verbunden sein. Der Indikator kann durch Drucken an der Paneelverpackung vorgesehen werden.

Zudem kann eine Abdeckung für den Indikator vorgesehen sein.

## Patentansprüche

1. Paneelverpackungsanordnung,
- mit einer Paneelverpackung mit einer Umhüllung,
- mit mindestens einem von der Umhüllung aufgenommenen Paneel (3) zum Verlegen auf einem Boden und
- mit mindestens einem an der Paneelverpackung angebrachten Indikator (5) zur Anzeige von mindestens einer Beanspruchung, die eine durch Temperatur und/oder Feuchtigkeit bedingte Beanspruchung ist, in der ferner
- mehrere Indikatoren (5) vorgesehen sind, die ständig in Abhängigkeit von der mindestens einen Beanspruchung ihr Erscheinungsbild ändern,
- die Umhüllung eine Verpackungsfolie (9) aufweist,
- ein Indikator (5) außen auf der Paneelverpackung aufgebracht ist und von außen auf die Paneelverpackung wirkende Beanspruchungen anzeigt und
- ein weiterer Indikator (5) an der Innenseite der Verpackungsfolie (9) oder an einem Verpackungskarton (11) der Umhüllung, der von der Verpackungsfolie (9) umschlossen ist, angebracht ist.

2. Paneelverpackungsanordnung nach Anspruch 1,
**dadurch gekennzeichnet,**
**dass** der Indikator (5) eine Abdeckung aufweist.

3. Paneelverpackungsanordnung nach Anspruch 1 oder 2,
**dadurch gekennzeichnet,**
**dass** mindestens eine das Erscheinungsbild des Indikators (5) erläuternde Skala (13) vorgesehen ist.

4. Paneelverpackungsanordnung nach einem der Ansprüche 1 bis 3,
**dadurch gekennzeichnet,**
**dass** der Indikator (5,15) auf der Verpackungsfolie (9) oder dem Verpackungskarton (11) als Befestigungselement aufgedruckt ist.

## Claims

1. A panel packaging arrangement,
- comprising a panel packaging comprising a wrapping,
- comprising at least one panel (3) accommodated by the wrapping for installation on a floor and
- comprising at least one indicator (5) attached to the panel packaging for displaying at least one stress, which is a stress caused by temperature and/or moisture,
in which furthermore
- a plurality of indicators (5) are provided, which permanently change their appearance as a function of the at least one stress,
- the wrapping encompasses a packaging film (9),
- one indicator (5) is attached on the outside of the panel packaging and displays stresses acting on the panel packaging from the outside and
- a further indicator (5) is attached to the inside of the packaging film (9) or to a packaging cardboard (11) of the wrapping, which is surrounded by the packing film (9).

2. The panel packaging arrangement according to claim 1,
**characterized in**
**that** the indicator (5) encompasses a cover.

3. The panel packaging arrangement according to claim 1 or 2,
**characterized in**
**that** at least one scale (13), which explains the appearance of the indicator (5), is provided.

4. The panel packaging arrangement according to one of claims 1 to 3,
**characterized in**
**that** the indicator (5, 15) is printed onto the packaging film (9) or the packaging cardboard (11) as fastening element.

## Revendications

1. Dispositif d'emballage de lambris,
- avec un emballage de lambris comportant une enveloppe,
- avec au moins un lambris (3) logé dans l'enveloppe, destiné à être posé sur un sol et
- avec au moins un indicateur (5) monté sur l'emballage de lambris, pour afficher au moins une contrainte qui est une contrainte due à une température et/ou à de l'humidité,
dans lequel par ailleurs
- plusieurs indicateurs (5) sont prévus dont l'apparence change en permanence en fonction de l'au moins une contrainte,
- l'enveloppe comporte au moins un film d'emballage (9),
- un indicateur (5) est monté à l'extérieur sur l'emballage de lambris et affiche des contraintes agissant par l'extérieur sur l'emballage de lambris et
- un indicateur supplémentaire (5) qui est entouré par le film d'emballage (9) est monté sur la face intérieure du film d'emballage (9) ou sur un carton d'emballage (11) de l'enveloppe.

2. Dispositif d'emballage de lambris selon la revendication 1,
**caractérisé en ce que**
l'indicateur (5) comporte un couvercle.

3. Dispositif d'emballage de lambris selon la revendication 1 ou 2,
**caractérisé en ce que**
au moins une graduation (13) expliquant l'au moins une apparence de l'indicateur (5) est prévu.

4. Dispositif d'emballage de lambris selon l'une quelconque des revendications 1 à 3,
**caractérisé en ce que**
en tant qu'élément de fixation l'indicateur (5, 15) est empreint sur le film d'emballage (9) ou sur le carton d'emballage (11).
